# EUROPEAN PATENT SPECIFICATION

(11) **EP 2 289 865 B1**
(45) Date of publication and mention of the grant of the patent: **07.06.2017**
(21) Application number: 10194845.3
(22) Date of filing: 22.12.2008
(51) Int. Cl.: C07C 17/25, C07C 17/354, C07C 17/383, C07C 19/08, C07C 21/18

(54) **Process for producing 1,1,1,2-tetrafluoropropene**
VERFAHREN ZUR HERSTELLUNG VON 1,1,1,2-TETRAFLUORPROPEN
PROCÉDÉ DE PRODUCTION DE 1,1,1,2-TÉTRAFLUOROPROPÈNE

(30) Priority: 27.12.2007 US 17052 P
(43) Date of publication of application: 02.03.2011
(62) Divisional of application: 08868518.5
(73) Proprietor: Arkema France, 92700 Colombes (FR)
(72) Inventor: SHIBANUMA, Takashi, Nishihitotsuya Settsu-shi Osaka 566-8585 (JP); TAKAHASHI, Kazuhiro, Nishihitotsuya Settsu-shi Osaka 566-8585 (JP)

(56) References cited:
- EP-A1- 0 962 439
- GB-A- 785 079
- US-A- 4 086 407
- US-A1- 2007 179 324

## Description

### Technical Field

The present invention relates to a process for producing 2,2,2,3-tetrafluoropropene.

### Background Art

CFCs (chlorofluorocarbons) and HCFCs (hydrochlorofluorocarbons) were conventionally used as refrigerants. Since these substances can cause depletion of the ozone layer, HFCs (hydrofluorocarbons), particularly HFC-125 (pentafluoroethane) and HFC-32 (difluoromethane) have widely been used as substitute refrigerants. However, HFC-125 and HFC-32 are potent global warming substances and cause concern of diffusion of these substances to adversely affect the global Warming. Although these substances are recovered from disused apparatuses for the purpose of preventing their diffusion and consequently the global warming, all of these substances cannot be recovered. Also, diffusion caused by leaking or the like cannot be neglected. Then, CO₂ and hydrocarbon compounds are studied, to be used as other substitute refrigerants. However, the CO₂ refrigerant has poor efficiency and requires a larger device, so that there are a lot of problems in reducing overall greenhouse gas emission including consumption energy of the device. Also, the hydrocarbon compounds have problems about safety due to their high combustibility.

Recently, intense interest has been shown towards, as a. substitute refrigerant being able to solve these problems, 1,1,1,2-tetrafluoropropene (or 2,3,3,3-tetrafluoropropene, CF₃CF=CH₂, hereinafter also referred to as "HFC-1234yf"), which is a. HFC of an olefin having a low warming coefficient,

Some processes for producing HFC-1234yf have hitherto been disclosed. Patent Document 1 listed below describes a process of using CF₃CH=CH₂ as a raw material, halogenating it and then subjecting to a dehydrofluorination reaction to obtain HFC-1234yf. Patent Document 2 listed below describes a process of hydrogenating a fluorinated olefin having 3 to 6 carbon, atoms through at least two reaction stages to give an alkane, and dehydrofluorinating the alkane to produce a fluorinated olefin such as HFC-1234yf. Patent Document 3 listed below describes a process of directly obtaining HFC-1234yf from a mixture of CF₃CF₂CHCl₂ (HCFC-225ca) and CClF₂CF₂CHClF (HCFC-225cb) using a Pd/C catalyst. Patent Document 4 listed below describes a process of coupling an alkane having 1 carbon atom with an alkane having 2 carbon atoms to produce an olefin having 3 carbon atoms such as HFC-1234yf, Patent Document 5 listed below describes a process of synthesizing an alkane having 3 carbon atoms by reacting an alkane having 1 carbon atom with an alkane having 2 carbon atoms, and dehyrohalogenating the synthesized alkane to produce a fluorinated olefin such as HFC-1234yf. Patent Document 6 listed below describes a process of passing CF₃CHFCHF2 (HFC-236ea) and CF₃CHFCH₂F (HFC-245eb) simultaneously through a catalyst layer and subjecting them dehydrofluorlnation to directly produce a mixture of HFC-1225ye (1,2,3,3,3-pentafluoropropene) and HFC-1234yf. Patent Document 7 listed below describes a process of fluorinating CX₃CXYCH₃ with HF concurrently with a dehydrofluorination reaction to produce HFC-1234yf. Patent document 9 discloses a process of polymerization which comprises (a) cyclodimerizing hexafluoropropylene and ethylene under dimerizing conditions at a temperature of 200°C to 600°C to form a codimer which is subsequently cracked to form an admixture of 2,3,3,3-tetrafluoropropene and vinyl chloride.
Patent Document 1: WO 2007/056194
Patent Document 2: U.S. Patent Application Publication No. 2007/0179324
Patent Document 3: WO 2007/086972
Patent Document 4; WO 2007/056127
Patent Document 5: WO 2007/056128
Patent Document 6: WO 2007/117391
Patent Document 7: WO 2007/056148
Patent Document 8: WO 93/25510
Patent document 9: US 4,086,407

### Disclosure of Invention

However, these processes may often produce, in addition to HFC-1234yf as the aimed product and HFC-245eb as a precursor thereof, by-products which cannot be converted into HFC-1234yf. An object of the present invention is to provide a process capable of producing HFC-1234yf with a higher selectivity than that of the conventional methods.

The present inventors have studied about use of hexafluoropropene (HFP) as a raw material for production of HFC-1234yf, also intensively studied about appropriate procedure for carrying out hydrogenation, dehydrofluorination and distillation, and finally accomplished the present invention.

In a first aspect of the present invention, there is provided a process for producing 2,2,3,3-tetrafluoropropene (HFC-1234yf), which comprises the steps of:
a) hydrogenating a raw material of hexafluoropropene (HFP) together with 1,2,3,3,3-pentafluoropropene (HFC-1225ye) in the presence of a reducing catalyst to obtain a reaction mixture containing 1,1,1,2,3,3-hexafluoropropane (HFC-236ea) and 1,1,1,2,3-pentafluoropropane (HFC-245eb);
b) subjecting the reaction mixture obtained from the step a) to a dehydrofluorination reaction to obtain a reaction mixture containing 1,2,3,3,3-pentafluoropropene (HFC-1225ye) and 2,3,3,3-tetrafluoropropene (HFC-1234yf);
c) subjecting the reaction mixture obtained from the step b) to distillation to separate into a 1A fraction containing 2,3,3,3-tetrafluoropropene (HFC-1234yf) and a 2A fraction containing 1,2,3,3,3-pentafluoropropene (HFC-1225ye); and
d) using 1,2,3,3,3-pentafluoropropene (HFC-1225ye) contained in the 2A fraction for the step a),
whereby 2,3,3,3-tetrafluoropropene is obtained in the 1A fraction.

It is considered that, in the process according to the first aspect of the present invention, for producing HFC-1234yf from HFP, the following reactions successively proceed via HFC-1225ye.

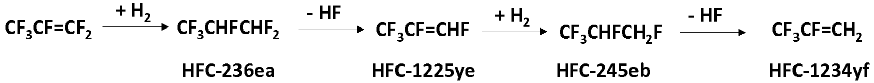

The present inventors have found that the hydrogenating reaction producing HFC-236ea from. HFP and the hydrogenating reaction producing HFC-245eb from HFC-1225ye can proceed under the same conditions, and also the dehydrofluorination reaction producing HFC-1225ye from HFC-236ea and the dehydrofluorination reaction producing HFC-1234yf from HFC-245eb can proceed under the same conditions. Thus, the hydrogenations and the dehydrofluorinations are respectively carried out in the same times (in other words, by "folding" the above successive reactions), and then distillation is carried out so that HFC-1234yf is obtained. According to this process of the present invention, it becomes possible to produce HFC-1234yf with a higher selectivity.

Furthermore, an apparatus for producing 2,3,3,3-tetrafluoropropene is provided and comprises :
a) hydrogenation reactor (R11),
b) an inlet for a starting material feed stream (F1), connected to said hydrogenation reactor, and
c) an outlet for hydrogenation reaction mixture (S11) connected to a dehydrofluorination reactor (R12),
d) an outlet for dehydrofluorination reaction mixture (S12) connected to a first distillation column (T11),
e) an outlet of first distillation column for high boiling point fraction 1A (S14) is connected to a second distillation column (T12),
f) an outlet (17) of second distillation column for recycling a stream (S16) comprising 1,2,3,3,3-pentafluoropropene to the hydrogenation reactor (R11),
g) an outlet (16) of second distillation column for recycling a stream (S15) comprising 1,2,3,3,3-pentafluoropropene and hexafluoropropène to the dehydrofluorination reactor (R12),
h) an outlet (14) of the first distillation column (T11) for final product stream (S13) of 2,3,3,3-tetrafluoropropene.

In the process according to the above first aspect of the present invention, the 2A fraction obtained from the step c) may contain, in addition to 1,2,3,3,3-pentafluoropropene (HFC-1225ye), at least one of 1,1,1,2,3-pentafluoropropane (HFC-245eb) and 1,1,1,2,3,3-hexafluoropropane (HFC-236ea); and the step d) may comprise subjecting the 2A fraction to further distillation to separate into a 3A fraction containing at least one of 1,1,1,2,3-pentafluoropropane (HFC-245eb) and 1,1,1,2,3,3-hexafluoropropane (HFC-236ea) and a 4A fraction containing 1,2,3,3,3-pentafluoropropene (HFC-1225ye), subjecting the 3A fraction to the dehydrofluorination reaction of the step b) and subjecting the 4A fraction to the hydrogenating reaction of the step a).

Therefore, at least one of HFC-245eb and HFC-236ea as intermediates and HFC-1225ye can be used by returning to the each appropriate reaction stage.

However, the first aspect of the present invention is not limited thereto. For example, all of the 2A fraction as is may be subjected to the hydrogenating reaction of the step a).

In a second aspect of the present invention, there is provided a process for producing 2,3,3,3-tetrafluoropropene (HFC-1234yf), which comprises the steps of:
p) hydrogenating a raw material of hexafluoropropene in the presence of a reducing catalyst to obtain a reaction mixture containing 1,1,2,3,3-hexafluoroproane (HFC-236ea) and 1,1,1,2,3-pentafluoropropane (HFC-245eb);
q) subjecting the reaction mixture obtained from the step p) to distillation to separate into a 1B fraction containing 1,1,1,2,3-pentafluoropropane (HFC-245eb) and a 2B fraction containing 1,1,1,2,3,3-hexafluoropropane (HFC-236ea); and r) subjecting the 1B fraction to a dehydrofluorination reaction to obtain a reaction mixture containing 2,3,3,3-tetrafluoropropene (HFC-1234yf),
   whereby 2,2,2,3-tetrafluoropropene (HFC-1234yf) is obtained in the reaction mixture of the step r).

It is considered that, in the process according to the second aspect of the present invention, for producing HFC-1234yf from HFP, the following reactions proceed.

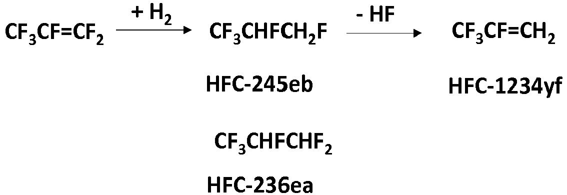

The present inventors have found that HFC-245eb is directly obtained, in addition to HFC-236ea, by hydrogenating HFP under appropriate conditions, and the resulting HFC-245eb is separated by distillation and is dehydrofluorinated to obtain HFC-1234yf. According to this process of the present invention, it becomes possible to produce HFC-1234yf with a higher selectivity.

The process according to the above second aspect of the present invention may further comprise supplying the 2B fraction obtained from the step q) to the hydrogenating reaction of the step p).

Therefore, HFC-236ea, as an intermediate can be subjected to hydrogenation to obtain HFC-245eb.

However, the second aspect of the present invention is not limited thereto. It is not required to the 2B fraction for hydrogenation.

According to the present invention, 2,3,3,3-tetrafluoropropene (HFC-1234yf) can be produced with a higher selectivity by using hexafluoropropene (HFP) as a raw material and appropriately combining hydrogenation, dehydrofluorination and distillation.

### Brief Description of Drawings

Fig. 1 shows a schematic diagram for explaining a process for producing 2,3,3,3-tetrafluoropropene (HFC-1234yf) in one embodiment of the present invention.
Fig. 2 shows a schematic diagram for explaining a process for producing 2,3,3,3-tetrafluoropropene (HFC-1234yf) which is not part of the present invention.

Following numerals denote the following elements: 11-17, 21-25...line; R11, R21...reactor (hydrogenating reaction); R12, R22... reactor (dehydrofluorination reaction); T11, T12, T21...distillation column; F1, F2...feed (mixture); S11...first stream (reaction mixture); S12... second stream (reaction mixture); S13... third stream (1A fraction); S14...fourth stream. (2A fraction); S15...fifth stream (3A fraction); S16...sixth stream (4A fraction); S21... first stream (reaction mixture); S22... second stream (1B fraction); S23... third stream (reaction mixture); and S24...fourth stream (2B fraction).

### Best Mode for Carrying Out the Invention

Two embodiments of the present invention will be described in detail below with reference to the drawings.

### (Embodiment 1)

The present embodiment relates to a manner in which following a hydrogenating reaction, dehydrofluorination is carried out, and then distillation is carried our ("folding" type).

Referring to Fig, 1, a raw material of hexafluoropropene (HFP) is fed from the outside to a line 11, and a. sixth stream (S16) taken out of a distillation column T12 through a line 17 as mentioned below is recycled to the line 11. The sixth: stream (S16) is a 4A fraction containing 1,2,3,3,3-pentafluoropropene (HFC-1225ye). Thus, the mixture of HFP and HFC-1225ye, as a feed (F1), is fed to a reactor R11 through the line 11,

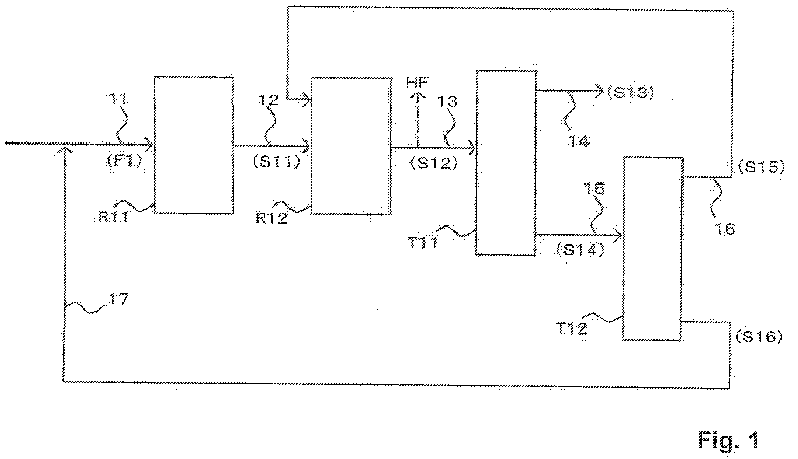

The mixture of HFP and HFC-1225ye fed as the feed (F1) is subjected to hydrogenation in the presence of a reducing catalyst, in the reactor R11 (in Fig. 1, supply of H₂ is omitted) to produce 1,1,1,2,3,3-hexafluoropropane (HFC-236ea) and 1,1,1,2,3-pentafluoropropane (HFC-245eb. In the reaction mixture, although the unreacted HFP and HFC-1225ye may slightly remain, it is preferred that there is substantially no material which would not be finally converted into HFC-1234yf.

As the reducing catalyst, for example, a general reducing catalyst can be used. A preferred reducing catalyst is a supported metal such as Pd, Pt, Rh, Ru or Re on activated carbon, a metal oxide such as alumina, or a metal fluoride. The ratio of metal supported is from 0.1 to 20 percent by weight, preferably from 0.1 to 10 percent by weight, and more preferably from 0.5 to 5 percent by weight.

Since ability of reduction varies depending on the kind of the catalyst and reduction may proceed excessively or may not proceed (a conversion ratio may not increase), the catalyst can be appropriately selected according to the purposes of the present embodiment.

The reaction pressure is not particularly limited and the reaction may proceed under reduced pressure, normal pressure or increased pressure, but the reaction under normal pressure or increased pressure is preferred. The pressure is preferably in a range from 0 to 1.5 MPaG (gauge pressure), since such pressure has an advantage in that it does not require a comparatively large device for proceeding the reaction. However, it is also possible to apply a pressure out of the above range.

The reaction temperature is usually in a range from 30 to 400 degrees centigrade, preferably from 30 to 300 degrees centigrade, and more preferably from 30 to 180 degrees centigrade The amount of by-products can be suppressed to a comparatively small amount by the reaction temperature not being unnecessarily high. Since this reaction is an exothermic reaction, a temperature higher than the above range may occur locally (at a part of the catalyst).

The contact time is represented by W/F0 (g·Nml⁻¹·sec) (in this case, F0 (Nml·sec⁻¹) denotes the amount of the feed (F1) supplied to the reactor R11, and W (g) denotes the amount of the catalyst filling the reactor R11), it may be usually in a range from, to 30, and is preferably in a range from 0.5 to 15 (the symbol "N" in the unit means conversion into a normal condition of 0 degrees centigrade and 1 atm, the same shall apply hereinafter). The contact time exerts an influence on selectivity and conversion ratio, and therefore can be appropriately selected according to the purposes.

Hydrogen (H₂) used for hydrogenation is usually mixed with HFP as the raw material and HFC-1225ye in a ratio not smaller than a theoretically equivalent amount and supplied to the reactor R11. Therefore, the molar ratio in supply of H₂/(HFP and HFC-1225ye) is usually in a range from 1 to 6, and more preferably from 1 to 3.

Then, the reaction mixture obtained in the reactor R11 is taken out as a first stream (S11) through a line 12 and supplied to a reactor R12. And, HFC-236ea and HFG-245eb in the reaction mixture are subjected to dehydrofluorination to produce 1,1,1,2,3-pentafluoropropene (HFC-1225ye) and 2,3,3,3-tetrafluoropropene (HFC-1234yf). In a reaction mixture thus obtained, unreacted HFC-236ea, HFC-245eb and so on may remain.

For the dehydrofluorination reaction, a catalyst which can be used for a general dehydrohalogenation reaction can be used. The catalyst is specifically, for example, a metal oxyfluoride or a metal fluoride, and more specifically chromium oxyfluoride, aluminum oxyfluoride, niobium fluoride, magnesium fluoride, tantalum fluoride or antimony fluoride, but is not particularly limited thereto. For another example, an activated carbon based catalyst as disclosed in Patent Document 8 listed above may also be used.

The reaction pressure is not particularly limited and the reaction may proceed under reduced pressure, normal pressure or increased pressure, but the reaction under normal pressure is preferred. The reaction under normal pressure is advantageous in comparison to the reaction under increased pressure in view of equilibrium, and has an advantage in comparison to the reaction under reduced pressure in that it does not require a comparatively large device.

The reaction temperature, in a case under normal pressure, can be in a range from 200 to 600 degrees centigrade, and preferably in a range from 250 to 450 degrees centigrade

The contact time is represented by W'/F0' (g·Nml⁻¹. sec) (in this case, F0' (Nml·sec⁻¹) denotes the amount of the first stream (S11) and, if any, a below-mentioned fifth stream (S15) supplied to the reactor R12, and W' (g) denotes the amount of the catalyst filling the reactor R12) may be usually in a range from 0.1 to 120, and is preferably in a range from 0.5 to 60.

In the reactions of the present embodiment, the dehydrofluorination reaction is a rate-limiting step.

Then, the reaction mixture obtained in the reactor R12 after dehydrofluorination, is taken out as a second stream (S12) through a line 13 and supplied to a distillation column T11. The second stream (S12) contains HFC-1234yf (normal boiling point of -29 degrees C) and HFC-1225ye (normal boiling points of -19.5 degrees centigrade (Z-isomer) and -15.3 degrees centigrade (E-isomer)) and is separated by distillation into a 1A fraction containing HFC-1234yf (low-boiling component) and a 2A fraction containing HFC-1225ye (high-boiling component).

The 1A fraction is taken out of the distillation column T11 as a third stream (S13) through a line 14. The 1A fraction is preferably composed of HFC-1234yf substantially, and by the 1A fraction, HFC-1234yf can be obtained.

The 2A fraction is taken out of the distillation column T11 as a fourth stream (S14) through a line 15. The 2A fraction may generally contain, in addition to HFC-1225ye, HFC-245eb (normal boiling point of 23 degrees C) and/or HFC-236ea (normal boiling point of 6 degrees C).

The 2A fraction thus obtained after taking out of the distillation column T11 as the fourth stream (S14), is supplied to a further distillation column T12 and separated by distillation into a 3A fraction containing at least one of, generally both of, HFC-245eb and HFC-236ea (high-boiling components) and a 4A fraction containing HFC-1225ye (low-boiling component). The 3A fraction is taken out of the distillation column T12 through a line 16 as a fifth stream (S15), and may be returned to the reactor R12, where it is subjected to the dehydrofluorination reaction. The 4A. fraction is taken out of the distillation column T12 through a line 17 as the sixth stream (S16), and supplied to the reactor R11 together with HFP as the feed (F1) as mentioned above, where it is subjected to the hydrogenating reaction.

As described above, according to the present embodiment, HFC-1234yf can be produced with a high selectivity by carrying out the successive reactions for obtaining HFC-1234yf from HFP through HFC-1225ye in the simplified process. The process of the present embodiment can be conducted continuously.

Although an amount of impurities produced in the present embodiment is quite small, low-boiling and high-boiling impurities may be removed from the stream(s) (e.g. the product stream and the recycling stream) by distillation, if appropriate. Hydrogen fluoride (HF) produced during the step may be separated and removed by washing with water and/or distillation, if appropriate. Although the unreacted hydrogen (H₂) may exist in the stream(s), it may be appropriately treated by, for example, separation and removal, or recycling to the reactor R11.

### (Embodiment 2 - not part of the present invention)

The present embodiment relates to a manner in which following a hydrogenating reaction, distillation is carried out, and then dehydrofluorination is carried out ("direct" type). Hereinafter, the present embodiment is similar to Embodiment 1 unless otherwise specified.

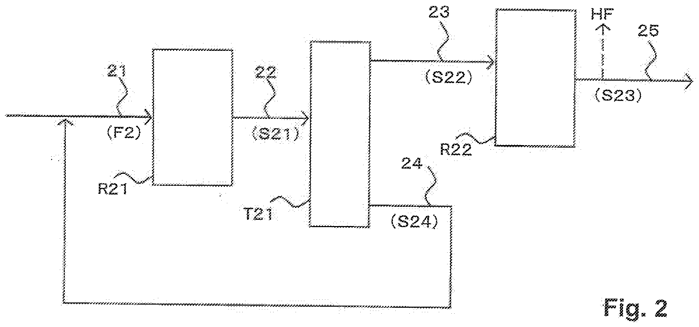

Referring to Fig. 2, a raw material of hexafluoropropene (HFP) is fed from the outside to a line 21 and a fourth stream (S24) taken out of a distillation column T21 through a line 24 as mentioned below is recycled to the line 21. The fourth stream (S24) is a 2B fraction containing HFC-236ea. Thus, the mixture of HFP and HFC-236ea, as a feed (F2), is fed to a R21 through the line 21. However, it should be noted that feed of HFC-236ea is not essential to the present embodiment.

The mixture of HFP and HFC-236ea fed as the feed (F2) is subjected to hydrogenation in the presence of a reducing catalyst in the reactor R21 (in Fig. 2, supply of H₂ is omitted) to produce HFC-236ea and HFC-245eb. In the present embodiment, even if only HFP is fed as the feed (F2), HFC-245eb can be produced directly from HFP. In the reaction mixture, HFC-1225ye may also be contained.

As the reducing catalyst, any appropriate reducing catalyst capable of directly producing HFC-245eb from HFP, for example, those mentioned in Embodiment 1 may be used accordingly.

Since ability of reduction varies depending on the kind of the catalyst and reduction may proceed excessively or may not proceed (a conversion ratio may not increase), the catalyst can be appropriately selected according to the purposes of the present embodiment.

The reaction pressure is not particularly limited and the reaction may proceed under reduced pressure, normal pressure or increased pressure, but the reaction under normal pressure or increased pressure is preferred. The pressure is preferably in a range from 0 to 1.5 MPaG (gauge pressure), since such pressure has an advantage in that it does not require a comparatively large device for proceeding the reaction. However, it is also possible to apply a pressure out of the above range.

The reaction temperature is usually in a range from 30 to 500 degrees centigrade, preferably from 30 to 400 degrees centigrade, and more preferably from 30 to 300 degrees centigrade The amount of by-products can be suppressed to a comparatively small amount by the reaction temperature not being unnecessarily high. Since this reaction is an exothermic reaction, a temperature higher than the above range may occur locally (at a part of the catalyst).

The contact time is represented by W/F0 (g·Nm⁻¹·sec) (in this case, F0 (Nml·sec⁻¹) denotes the amount of the feed (F2) supplied to the reactor R21, and W (g) denotes the amount of the catalyst filling the reactor R21), it may be usually in a range from 0.1 to 30, and is preferably in a range from 0.5 to 15. The contact time exerts an influence on selectivity and conversion ratio, and therefore can be appropriately selected according to the purposes.

Hydrogen (H₂) used for hydrogenation is usually mixed with HEP as the raw material (and HFC-236ea when it exists in the feed as in the present embodiment) in a ratio not smaller than a theoretically equivalent amount and supplied to the reactor R21. Therefore, the molar ratio in supply (which corresponds to the volume ratio in a gaseous state) of H_{2/}/HFP (or H₂/(HFP and HFC-236ea) when HFC-236ea exists in the feed as in the present embodiment) is usually in a range from 1 to 6, and more preferably from 1 to 4.

Then, the reaction mixture obtained in the reactor R21 is taken out as a first stream (S21) through a line 22 and supplied to a distillation column T21. The first stream (S21) contains HFC-245eb and HFC-236ea and is separated by distillation into a 1B fraction containing HFC-245eb (high-boiling component) and a 2B fraction containing HFC-236ea (low-boiling component).

The 1B fraction is taken out of the distillation column T21 through a line 23 as a second stream (S22) and supplied to a reactor R22. And, HFC-245eb in the 1B fraction is subjected to dehydrofluorination to produce HFC-1234yf.

The conditions of the dehydrofluorination reaction are the same as those of Embodiment 1.

The reaction pressure is not particularly limited and the reaction may proceed under reduced pressure, normal pressure or increased pressure, but the reaction under normal pressure is preferred. The reaction under normal pressure is advantageous in comparison to the reaction under increased pressure in view of equilibrium, and has an advantage in comparison to the reaction under reduced pressure in that it does not require a comparatively large device.

The reaction temperature, in a case under normal pressure, can be in a range from 200 to 600 degrees centigrade, and preferably in a range from 250 to 450 degrees centigrade

The contact time is represented by W'/F0' (g·Nml⁻¹·see) (in this case, F0' (Nml·sec⁻¹) denotes the amount of the second stream (S22) and, if any, the below-mentioned recycling stream supplied to a reactor R22, and W' (g) denotes the amount of the catalyst filling the reactor R22), it may be usually in a range from 0.1 to 120, and is preferably in a range from 0.5 to 60.

Although the unreacted HFC-245eb may also remain, the reaction mixture thus obtained preferably contains HFC-1234yf in high purity. Therefore, HFC-1234yf can be obtained. If desired, the reaction mixture may be separated into a fraction containing HFC-1234yf and a fraction containing HFC-245eb, and the latter fraction may be returned (recycling stream) to the reactor R22, where it is subjected to the dehydrofluorination reaction. Also in the reactions of the present embodiment, the dehydrofluorination reaction is a rate-limiting step.

On the other hand, the 2B fraction is taken out of the distillation column T21 through a line 24 as the fourth stream (S24), and supplied to the reactor R21 together with HFP as the feed (F2) as mentioned above, where it is subjected to the hydrogenating reaction. The 2B fraction may generally contain HFC-1225ye, in addition to HFC-236ea.

As described above, according to the present embodiment, HFC-1234yf can be produced with a high selectivity by the very simple process which utilizes the reaction capable of directly obtaining HFC-245eb from HFP. The process of the present embodiment can also be conducted continuously.

Although an amount of impurities produced in the present embodiment is quite small, low boiling and high boiling impurities may be removed from the stream(s) (e.g. the product stream and the recycling stream) by distillation if appropriate. Hydrogen fluoride (HF) produced during the step may be separated and removed by washing with water and/or distillation, if appropriate. Although the unreacted hydrogen (H₂) may exist in the stream(s), it may be appropriately treated by, for example, separation and removal, or recycling to the reactor R21.

### [Example 1]

The process of the present invention was carried out in accordance with Embodiment 1 described in detail with reference to Fig. 1.

As a raw material, HFP was used in a gaseous state. This raw material and a sixth stream (S16) recycled from the distillation column T12 were combined and then fed, as a feed (F1) to the reactor R11, where the feed was subjected to the hydrogenation reaction in the presence of a reducing catalyst. As the reducing catalyst, an activated carbon-supported Pd catalyst (ratio of Pd supported: 3 percent by weight) was used. The reaction conditions were as follows: The reaction temperature was 200 degrees centigrade, and the reaction pressure was normal pressure. The volume ratio in supply of H₂/(HFP and HFC-1225ye) was adjusted to 3. W/F0 = 8 (g·Nml⁻¹·sec), wherein F0 (Nml·sec⁻¹) denotes the amount of the feed (F1) supplied to the reactor R11, and W (g) denotes the amount of the catalyst filling the reactor R11.

The reaction mixture obtained in the reactor R11 was taken out as a first stream (S11) and supplied to the reactor R12, where it was subjected to the dehydrofluorination reaction. A fifth stream (S15) recycled from the distillation column T12 was also supplied to the reactor R12, and subjected to the dehydrofluorination reaction. As the catalyst., a chromium oxyfluoride catalyst (fluoride content: 31.4 percent by weight) was used. The reaction conditions were as follows: The reaction temperature was 400 degrees centigrade, and the reaction pressure was normal pressure. W'/F0' = 20 (g·Nml⁻¹.sec), wherein F0' (Nml.sec⁻¹) denotes the amount of the first stream (S11) and the fifth stream (S15) supplied to the reactor R12, and W' (g) denotes the amount of the catalyst filling the reactor R12.

The reaction mixture obtained in the reactor R12 after the dehydrofluorination was taken, out as a second stream (S12) and transferred to the distillation column where it was separated into a third stream (S13) as a low-boiling part and a fourth stream (S14) as a high-boiling part. The resultant fourth stream (314) was transferred to the distillation column T12, where it was separated into a fifth stream (S15) as a high-boiling part and a sixth stream (S16) as a low-boiling part. The resultant fifth stream (S15) and sixth stream (S16) were recycled entirely to the reactor R12 and the reactor R11 respectively, as described above.

Twelve hours after starting the operation, compositions of the feed (F1) and the first to sixth streams (S11 to S16) were analyzed by gas chromatography to determine the flow rate of each component excluding hydrogen (H₂) and hydrogen fluoride (HF). The results are shown in Table 1. In the table, the symbol "-" means no detection, and "trace" means existence only in a trace amount.

**Table 1**

| | Flow rate (kmol/hr) | | | | | | |
|---|---|---|---|---|---|---|---|
| | F1 | S11 | S12 | S13 | S14 | S15 | S16 |
| HFP | 0.51 | - | - | - | - | - | - |
| HFC-236ea | - | 0.51 | 0.08 | - | 0.08 | 0.08 | - |
| HFC-245eb | - | 0.49 | 0.05 | - | 0.05 | 0.05 | - |
| HFC-1234yf | - | - | 0.50 | 0.50 | - | - | - |
| HFC-1225ye | 0.49 | - | 0.49 | - | 0.49 | - | 0.49 |
| Impurities | trace | trace | 0.02 | 0.01 | 0.01 | 0.01 | trace |

It is confirmed from Table 1 that HFC-1234yf can be obtained as the third stream (S13), which corresponds to the 1A fraction, with a high yield.

### [Example 2]

The process of the present invention was carried out in accordance with Embodiment 2 described in detail with reference to Fig. 2.

As a raw material, HFP was used in a gaseous state. This raw material and a fourth stream (S24) recycled from the distillation column T21 were combined and then fed, as a feed (F2), to the reactor R21, where the feed was subjected to the hydrogenation reaction in the presence of a reducing catalyst. As the reducing catalyst, an activated carbon-supported Pd catalyst (ratio of Pd supported: 3 percent by weight) was used. The reaction conditions were as follows: The reaction temperature was 200 degrees centigrade, and the reaction pressure was normal pressure. The volume ratio in supply of H₂/(HFP and HFC-236ea) was adjusted to 4. W/F0 === 8 (g·Nml⁻¹·sec), wherein F0 (Nml.sec⁻¹) denotes the amount of the feed (F2) supplied to the reactor R21, and W (g) denotes the amount of the catalyst filling the reactor R21.

The reaction mixture obtained in the reactor R21 was taken out as a first stream (S21) and supplied to the distillation column T21, where it was separated into a second stream, (S22) as a high-boiling part and a fourth stream (S24) as a low-boiling part.

The resulting second stream (S22) was supplied to the reactor R22, where it was subjected to the dehydrofluorination reaction. As the catalyst, a chromium oxyfluoride catalyst (fluorine content: 31.4 percent by weight) was used. The reaction conditions were as follows: The reaction temperature was 400 degrees centigrade, and the reaction pressure was normal pressure, W'/F0' = 20 (g·Nml⁻¹·sec), wherein F0' (Nml·sec⁻¹) denotes the amount of the second stream (S22) supplied to the reactor R22, and W' (g) denotes the amount of the catalyst filling the reactor R22.

On the other hand, the fourth stream (S24) was recycled entirely to the reactor 21, as described above.

Twelve hours after starting the operation, compositions of the feed (F2) and the first to fourth streams (S21 to S24) were analyzed by gas chromatography to determine the flow rate of each component excluding hydrogen (H₂) and hydrogen fluoride (HF). The results are shown in Table 2, In the table, the symbol "-" means no detection, and "trace" means existence only in a trace amount.

**Table 2**

| | Flow rate (kmol/hr) | | | | |
|---|---|---|---|---|---|
| | F2 | S21 | S22 | S23 | S24 |
| HFP | 0.2 | - | - | - | - |
| HFC-1225ye | trace | trace | - | - | trace |
| HFC-236ea | 0.9 | 0.9 | - | - | 0.9 |
| HFC-245eb | - | 0.2 | 0.2 | 0.016 | - |
| HFC-1234yf | - | - | - | 0.18 | - |
| Impurities | trace | trace | trace | 0.004 | trace |

It is confirmed from Table 2 that HFC-1234yf can be obtained in the third stream (S23), which corresponds to the reaction mixture after dehydrofluorination, in a yield of 90 percent. Since HFC-245eb in the third stream is an intermediate, a high yield of 97 percent or more can be expected when HFC-245eb is separated and recycled to the dehydrofluorinated reaction to obtain HFC-1234yf.

### Industrial Applicability

2,3,3,3-tetrafluoropropene (HFC-1234yf) can be used as a refrigerant substance, and a process for producing HFC-1234yf using hexafluoropropene (HFP) as a raw material can be expected to be useful as a process for producing a substitute refrigerant.

## Claims

1. An apparatus for producing 2,3,3,3-tetrafluoropropene comprising :
a) hydrogenation reactor (R11),
b) an inlet for a starting material feed stream (F1), connected to said hydrogenation reactor, and
c) an outlet for hydrogenation reaction mixture (S11) connected to a dehydrofluorination reactor (R12),
d) an outlet for dehydrofluorination reaction mixture (S12) connected to a first distillation column (T11),
e) an outlet of first distillation column for high boiling point fraction 1A (S14) is connected to a second distillation column (T12),
f) an outlet (17) of second distillation column for recycling a stream (S16) comprising 1,2,3,3,3-pentafluoropropene to the hydrogenation reactor (R11),
g) an outlet (16) of second distillation column for recycling a stream (S15) comprising 1,2,3,3,3-pentafluoropropene and hexafluoropropene to the dehydrofluorination reactor (R12),
h) an outlet (14) of the first distillation column (T11) for final product stream (S13) of 2,3,3,3-tetrafluoropropene.

2. A process for producing 2,3,3,3-tetrafluoropropene comprising :
a) hydrogenating a raw material of hexafluoropropene together with 1,2,3,3,3-pentafluoropropene in the presence of a reducing catalyst to produce 1,1,1,2,3,3-hexafluoropropane and 1,1,1,2,3-pentafluoropropane,
b) dehydrofluorinating the hexafluoropropane and pentafluoropropane in step (a) in the presence of a catalyst to produce a reaction mixture of 1,1,1,2,3-pentafluoropropene and 2,3,3,3-tetrafluoropropene,
c) the reaction mixture of (b) is supplied to a first distillation column to obtain a stream comprising substantially 2,3,3,3-tetrafluoropropene and a stream 2A comprising of 1,2,3,3,3-pentafluoropropene, 1,1,1,2,3-pentafluoropropane and 1,1,1,2,3,3-hexafluoropropane,
d) the stream 2A in step (c) is supplied to a second distillation column to obtain a stream 3A containing at least one of 1,1,1,2,3-pentafluoropropane and 1,1,1,2,3,3-hexafluoropropane which is recycled to step (b) and a stream 4A containing 1,2,3,3,3-pentafluoropropene which is recycled to step (a).

3. Process according to claim 2 **characterised in that** the temperature of hydrogenation is between 30 to 300°C, preferably 30 to 180°C.

4. Process according to claim 2 or 3 **characterised in that** the temperature of dehydrofluorination is between 200 to 600°C, preferably 250 to 450°C.

## Patentansprüche

1. Vorrichtung zur Herstellung von 2,3,3,3-Tetrafluorpropen, die Folgendes aufweist:
a) einen Hydrierreaktor (R11),
b) einen Einlass für einen Ausgangsstoff-Einsatzstrom (F1), der mit dem Hydrierreaktor verbunden ist, und
c) einen Auslass für eine Hydrierungsreaktionsmischung (S11), der mit einem Dehydrofluorierungsreaktor (R12) verbunden ist,
d) einen Auslass für eine Dehydrofluorierungsreaktionsmischung (S12), der mit einer ersten Destillationssäule (T11) verbunden ist,
e) einen Auslass der ersten Destillationssäule für eine hochsiedende Fraktion 1A (S14), der mit einer zweiten Destillationssäule (T12) verbunden ist,
f) einen Auslass (17) der zweiten Destillationssäule zur Rückführung eines 1,2,3,3,3-Pentafluorpropen umfassenden Stroms (S16) zum Hydrierreaktor (R11),
g) einen Auslass (16) der zweiten Destillationssäule zur Rückführung eines 1,2,3,3,3-Pentafluorpropen und Hexafluorpropen umfassenden Stroms (S15) zum Dehydrofluorierungsreaktor (R12),
h) einen Auslass (14) der ersten Destillationssäule (T11) für einen Endproduktstrom (S13) von 2,3,3,3-Tetrafluorpropen.

2. Verfahren zur Herstellung von 2,3,3,3-Tetrafluorpropen, das Folgendes umfasst:
a) Hydrieren eines Ausgangsstoffs von Hexafluorpropen zusammen mit 1,2,3,3,3-Pentafluorpropen in Gegenwart eines reduzierenden Katalysators zur Herstellung von 1,1,1,2,3,3-Hexafluorpropan und 1,1,1,2,3-Pentafluorpropan,
b) Dehydrofluorieren des Hexafluorpropans und Pentafluorpropans in Schritt (a) in Gegenwart eines Katalysators zur Herstellung einer Reaktionsmischung von 1,1,1,2,3-Pentafluorpropen und 2,3,3,3-Tetrafluorpropen,
c) die Reaktionsmischung von (b) wird einer ersten Destillationssäule zugeführt, wobei man einen Strom, der weitgehend 2,3,3,3-Tetrafluorpropen umfasst, und einen Strom 2A, der 1,2,3,3,3-Pentafluorpropen, 1,1,1,2,3-Pentafluorpropan und 1,1,1,2,3,3-Hexafluorpropan umfasst, erhält,
d) der Strom 2A in Schritt (c) wird einer zweiten Destillationssäule zugeführt, wobei man einen 1,1,1,2,3-Pentafluorpropan und/oder 1,1,1,2,3,3-Hexafluorpropan enthaltenden Strom 3A, der zu Schritt (b) zurückgeführt wird, und einen 1,2,3,3,3-Pentafluorpropen enthaltenden Strom 4A, der zu Schritt (a) zurückgeführt wird, erhält.

3. Verfahren nach Anspruch 2, **dadurch gekennzeichnet, dass** die Hydrierungstemperatur zwischen 30 bis 300°C, vorzugsweise 30 bis 180°C, liegt.

4. Verfahren nach Anspruch 2 oder 3, **dadurch gekennzeichnet, dass** die Dehydrofluorierungstemperatur zwischen 200 bis 600°C, vorzugsweise 250 bis 450°C, liegt.

## Revendications

1. Appareil de production de 2,3,3,3-tétrafluoropropène comprenant :
a) un réacteur d'hydrogénation (R11),
b) une entrée pour un flux d'alimentation de matériau de départ (F1), raccordée audit réacteur d'hydrogénation, et
c) une sortie pour mélange de réaction hydrogénation (S11) raccordée à un réacteur de déshydrofluoration (R12),
d) une sortie pour mélange de réaction de déshydrofluoration (S12) raccordée à une première colonne de distillation (T11),
e) une sortie de première colonne de distillation pour fraction à point d'ébullition élevé 1A (S14) est raccordée à une deuxième colonne de distillation (T12),
f) une sortie (17) de deuxième colonne de distillation pour recycler un flux (S16) comprenant du 1,2,3,3,3-pentafluoropropène vers le réacteur d'hydrogénation (R11),
g) une sortie (16) de deuxième colonne de distillation pour recycler un flux (S15) comprenant du 1,2,3,3,3-pentafluoropropène et de l'hexafluoropropène vers le réacteur de déshydrofluoration (R12),
h) une sortie (14) de la première colonne de distillation (T11) pour le flux de produit final (S13) de 2,3,3,3-tétrafluoropropène.

2. Procédé de production de 2,3,3,3-tétrafluoropropène comprenant :
a) l'hydrogénation d'une matière première d'hexafluoropropène conjointement avec du 1,2,3,3,3-pentafluoropropène en présence d'un catalyseur de réduction pour produire du 1,1,1,2,3,3-hexafluoropropane et du 1,1,1,2,3-pentafluoropropane,
b) la déshydrofluoration de l'hexafluoropropane et du pentafluoropropane de l'étape (a) en présence d'un catalyseur pour produire un mélange de réaction de 1,1,1,2,3-pentafluoropropène et de 2,3,3,3-tétrafluoropropène,
c) le mélange de réaction de (b) est alimenté dans une première colonne de distillation pour obtenir un flux comprenant sensiblement du 2,3,3,3-tétrafluoropropène et un flux 2A constitué de 1,2,3,3,3-pentafluoropropène, 1,1,1,2,3-pentafluoropropane et 1,1,1,2,3,3-hexafluoropropane,
d) le flux 2A dans l'étape (c) est alimenté dans une deuxième colonne de distillation pour obtenir un flux 3A contenant au moins l'un du 1,1,1,2,3-pentafluoropropane et du 1,1,1,2,3,3-hexafluoropropane qui est recyclé vers l'étape (b) et un flux 4A contenant du 1,2,3,3,3-pentafluoropropène qui est recyclé vers l'étape (a).

3. Procédé selon la revendication 2 **caractérisé en ce que** la température d'hydrogénation est comprise entre 30 et 300 °C, de préférence entre 30 et 180 °C.

4. Procédé selon la revendication 2 ou 3 **caractérisé en ce que** la température de déshydrofluoration est comprise entre 200 et 600 °C, de préférence entre 250 et 450 °C.
